# EUROPEAN PATENT SPECIFICATION

(11) **EP 2 575 918 B1**
(45) Date of publication and mention of the grant of the patent: **14.05.2014**
(21) Application number: 11725257.7
(22) Date of filing: 26.05.2011
(51) Int. Cl.: A61F 9/007, A61M 1/00

(54) **Ophthalmic surgery pump system comprising a surgical cassette with an identification feature**
Augenchirurgisches Pumpsystem mit einer chirurgischen Kassette mit einem Identifikationsmerkmal
Pompe pour chirurgie ophthalmique comprenant une cassette chirurgicale avec un élément d'identification

(30) Priority: 26.05.2010 US 348407 P; 21.04.2011 US 201113091279
(43) Date of publication of application: 10.04.2013
(73) Proprietor: Bausch & Lomb Incorporated, Rochester, NY 14604-2701 (US)
(72) Inventor: PERKINS, James, T., St. Charles, MO 63304 (US); HERTWECK, David, W., Valley Park, MO 63088 (US)
(74) Representative: Vossius & Partner
(86) International application number: PCT/US2011/038025
(87) International publication number: WO 2011/150141

(56) References cited:
- EP-A1- 0 777 111
- WO-A2-2007/143677
- US-A- 5 647 854
- US-A- 5 899 674

## Description

### BACKGROUND

### Field

The present invention is directed to an ophthalmic surgery pump system for use during an ophthalmic surgery procedure. More specifically, the present disclosure is directed towards an ophthalmic surgical cassette having a removable manifold base, including an identification feature for indicating a cassette type to an ophthalmic surgical console.

### Description of the Related Art

The statements in this section merely provide background information related to the present disclosure and may not constitute prior art.

Ophthalmic surgical cassettes for use with pump systems during ophthalmic surgical procedures are generally known. Ophthalmic surgical cassettes commonly include a container for retaining aspirant fluid and tissue retrieved from a surgical site during an ophthalmic surgical procedure. It is known for ophthalmic surgical cassettes to also include a fluid level indicator, which indicates to a surgeon when an ophthalmic surgical cassette has retained a pre-determined volume of aspirant fluid and tissue. Further, a variety of identification schemes are similarly known in the art to signal to a type of ophthalmic surgical cassette.

In WO 2007/143677 A2, a cassette for use with an ophthalmic surgical pump for collecting aspirant fluid and tissue from a patient's eye is disclosed, the cassette including a rigid walled container having an interior volume, wherein at least one tapered alignment slot is formed in a side wall of the container and extends from a back wall towards a front wall, an irrigation and aspiration manifold base is removably attached to the container, an aspiration path is formed within the container for receiving the aspiration fluid and the tissue from the eye and directing the fluid flow towards a front half of the container before the fluid and tissue collects within a majority of the interior volume of the container, and a fluid level indicator is formed on a wall of the container such that an associated photo-detector of the pump may determine a level of fluid in the container. Furthermore, the fluid level indicator may include a geometric figure, which can be used to identify the type of cassette to be used in surgery.

US 5,899,674 A discloses a cassette having a series of break-off prismatic tabs integrally formed in the cassette that can be optically sensed by a surgical instrument.

US 5,647,854 A concerns a pump provided with a control module and an attachable fluid reservoir cassette, the control module including a pumping mechanism for pumping fluid from the fluid reservoir to the patient with the cassette being provided with appropriate indicia to identify differences between a plurality of cassettes.

There exists the need for an ophthalmic surgical cassette with an improved identification feature for indicating a cassette type to an ophthalmic surgical console. The present invention is defined by the claims.

### Brief Description of the Drawings

The drawings described herein are for illustration purposes only and are not intended to limit the scope of the present disclosure in any way.
FIG. 1 is a perspective view of an ophthalmic surgical cassette, according to the present disclosure;
FIG. 2 is a perspective view of an ophthalmic surgery pump system, including the ophthalmic surgical cassette of FIG. 1, according to the present disclosure;
FIG. 3 is a partial perspective view of the identification feature of the ophthalmic surgical cassette of FIG. 1;
FIG. 4 is a graphical illustration of the refraction of light by the identification feature of the ophthalmic surgical cassette of FIG. 1;
FIG. 5 is an illustration of the light waveform detected from the ophthalmic surgical cassette of FIG. 1;
FIG. 6 is a partial perspective view of an identification feature, according to one embodiment of the present disclosure;
FIG. 7 is an illustration of the light waveform detected from an ophthalmic surgical cassette, including at least one treated surface; and
FIG. 8 is a perspective view of a manifold base, including an aspiration tube and an auxiliary aspiration tube, according to the present disclosure.

### Detailed Description of the Preferred Embodiment

The following description is merely exemplary in nature and is not intended to limit the present disclosure, application, or uses.

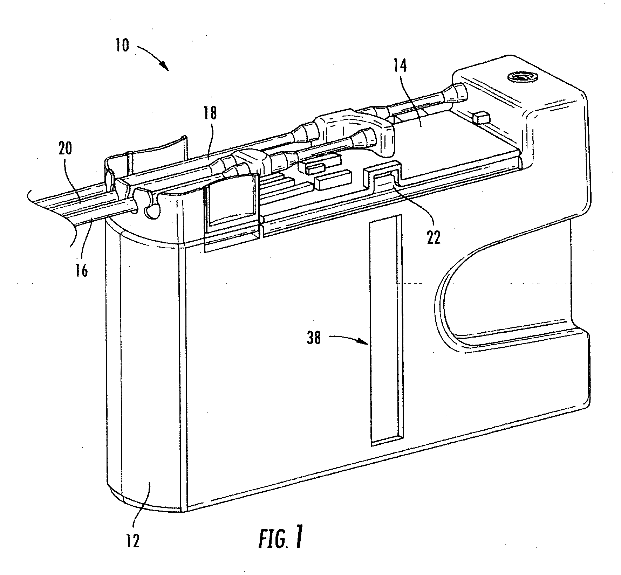

According to one illustrative example of the present disclosure, an ophthalmic surgical cassette 10 is illustrated in FIG. 1. The ophthalmic surgical cassette 10, includes a rigid-walled container 12, having an interior volume for collecting aspirant fluid and/or tissue, and a manifold base 14, including an irrigation tube 16 for coupling an irrigation source (not shown), and an aspiration tube 18 for coupling an ophthalmic surgical handpiece (not shown). The manifold base 14 is removeably attached to the rigid-walled container 12, such that the ophthalmic surgical cassette 10 may be emptied during an ophthalmic surgery procedure, without disconnecting the irrigation tube 16 from the irrigation source or the aspiration tube 18 from the ophthalmic surgical handpiece. The manifold base 14, further includes an irrigation tube 20 for providing irrigation to a surgical site.

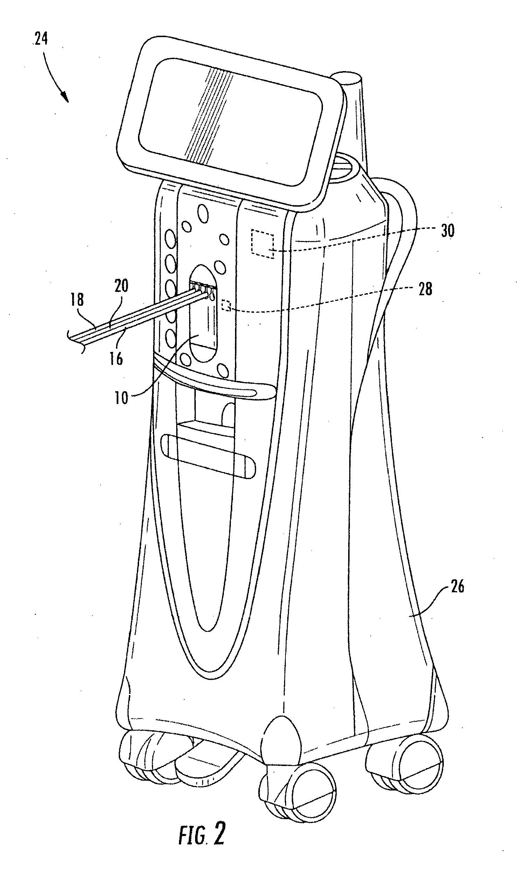

The manifold base 14 also includes an identification feature 22 for indicating a cassette type to an ophthalmic surgical console. In this example, the cassette type indicated by the identification feature 22 is anterior. An example of an ophthalmic surgical console is illustrated in FIG. 2 and described below. During an ophthalmic surgical procedure (except when being emptied), the ophthalmic surgical cassette 10 is disposed at least partially within an ophthalmic surgical console. The cassette type indicates to the ophthalmic surgical console which of one or more ophthalmic surgical procedures the ophthalmic surgical cassette 10 is configured to perform. Each ophthalmic surgical procedure may include multiple surgical functions, which the ophthalmic surgical console enables upon detecting the identification feature. In this manner, only the surgical functions usable by a particular cassette type are enabled for use by a surgeon, while other surgical functions may be disabled. Surgical functions may be enabled/disabled individually or associated with a surgical routine, such that all surgical functions in the surgical routine are enabled/disabled together, or some combination thereof. For example, when a cassette type is anterior, as in FIG. 1, an ophthalmic surgical console may disable surgical routines for posterior and/or combined (anterior and posterior) ophthalmic surgical procedures.

In one example, when a posterior cassette type is indicated by the identification feature of an ophthalmic surgery cassette, a surgical function potentially unused during a posterior procedure, *e.g.,* phacoemulsification etc., may be disabled by the ophthalmic surgical console. In at least one illustrative example of the present disclosure, a user may be forced to remove an incorrect ophthalmic surgical cassette and install a correct ophthalmic surgical cassette before an infended ophthalmic surgical procedure may be performed. Thus, an identification feature according to the present disclosure may ensure use of an ophthalmic surgical cassette in an appropriate ophthalmic surgical procedure, providing a potential safety feature.

In various examples of the present disclosure, the types and arrangements of tubes on a manifold base of an ophthalmic surgical cassette, in addition to other components, *e.g*., a reflux bulb, etc., often dictate which types of ophthalmic surgical procedures may be completed using said ophthalmic surgical cassette. Accordingly, an identification feature included in the manifold base, as compared to other components of an ophthalmic surgery cassette, provides a more accurate indication of the one or more surgical procedures for which the ophthalmic surgical cassette is intended. Further, when a manifold base is removable, *e.g*., removable manifold base 14 illustrated in FIG. 1, etc., the manifold base may be configured to receive a generic or universal size of container for convenience in manufacturing. If an indication scheme were implemented on a generic container or a container with a generic interface to a manifold base, containers may be mixed up and coupled to a manifold base configured for different types of ophthalmic surgical procedures, thereby providing an errant indication to an ophthalmic surgical console. As a result, the ophthalmic surgical console may disable surgical functions useable with the ophthalmic surgical cassette and/or the manifold base, and enable surgical functions not useable with the ophthalmic surgical cassette and/or the manifold base. Remedying the errant indication generally requires identification of the ophthalmic surgical cassettes, including swapped container (not always an easily visible indicator), and un-swapping the container. According to the present disclose, an identification feature included in a manifold base of an ophthalmic surgical cassette eliminates at least this potential error, even when a generic rigid-walled container is included.

FIG. 2 illustrates an embodiment of an ophthalmic surgery pump system 24, according to the present disclosure. The ophthalmic surgery pump system 24 is an ophthalmic surgical system for anterior, posterior, or combined procedures, including an ophthalmic surgical console 26 and the ophthalmic surgical cassette 10 installed in the ophthalmic surgical console 26.

The ophthalmic surgical console 26 includes a detector 28 disposed internal to the ophthalmic surgical console 26 (shown in broken lines) and adjacent to the ophthalmic surgical cassette 10 for detecting the identification feature 22. In this particular embodiment, the detector 28 includes a light source and a linear array of charged coupled devices (CCD). In other embodiments, a different arrangement of CCDs may be employed depending on a configuration of an ophthalmic surgical cassette and/or an ophthalmic surgical console. In still other configurations not falling within the scope of the claims, a detector may include a photo-detector, such as a CMOS device, a contact image sensor, or a different suitable device, etc., for detecting various types of identification features, through light transmission and/or otherwise. A detector may be disposed in one or more different locations, *e.g*., internal and/or external to an ophthalmic surgical console, etc., as long as the detector is disposed for detecting an identification feature included in a manifold base of an ophthalmic surgical cassette.

The detector 28 is operably coupled to a processor 30 (shown in broken lines) disposed internal to the ophthalmic surgical console 26. The processor 30 is operably coupled to the detector 28, such that when the detector 28 detects an identification feature, the processor 30 receives an input from the detector 28, and determines a cassette type of an ophthalmic surgical cassette installed in the ophthalmic surgery pump system 24. Then, the processor 30 may enable and/or disable surgical functions/routines appropriate to the installed ophthalmic surgical cassette. It should be appreciated that a processor may include a number of different processing devices, as well known in the art. For example, a processor may include a microprocessor, CPU, microcontroller, gate array, logic circuit, or one or more other suitable devices, etc.

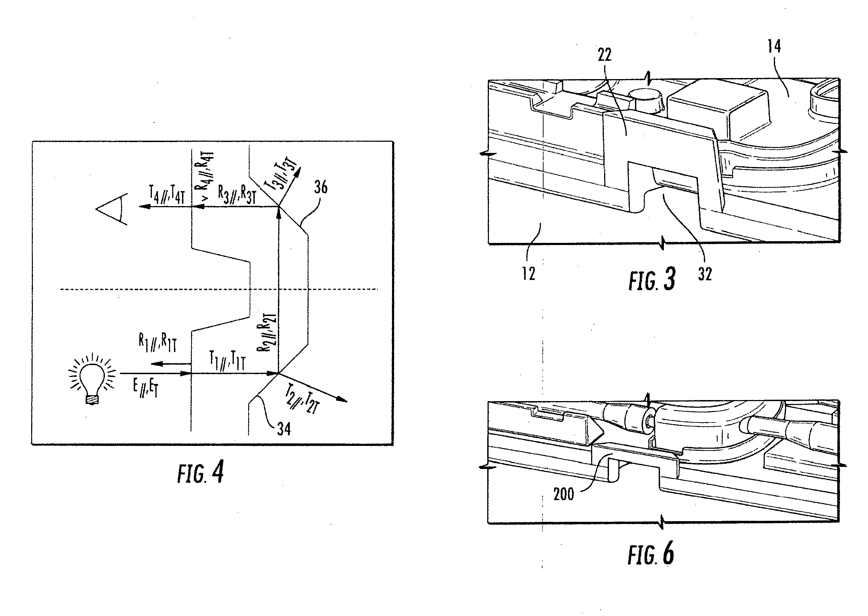

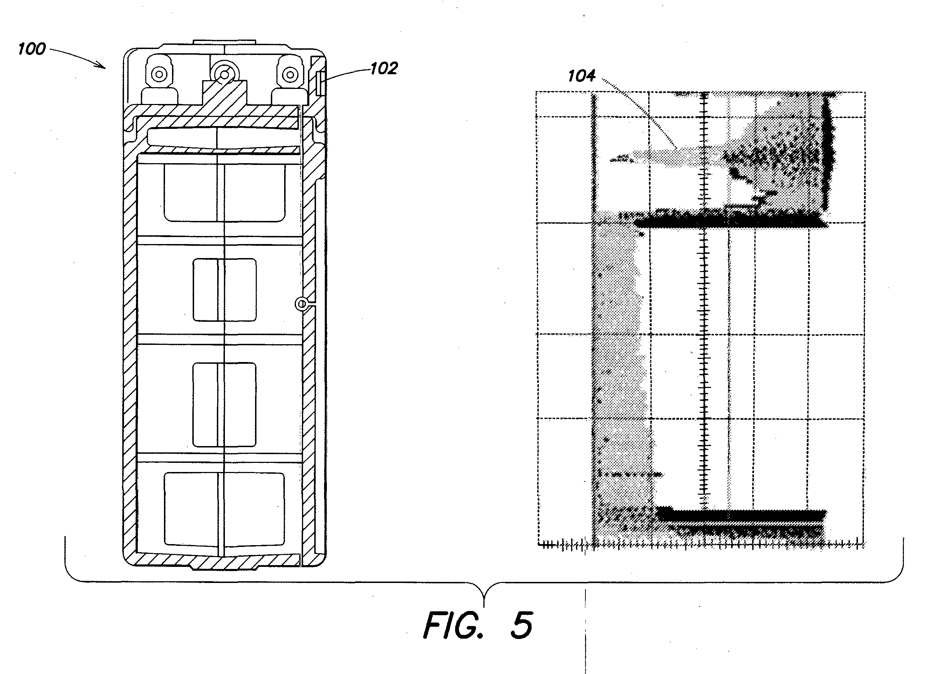

As shown in the detailed view of FIG. 3, the identification feature 22 includes a refraction element for refracting light transmission generated from the ophthalmic surgical console 26. The refraction element includes a notch section 32, forming a prism. Light transmission relative to the identification feature 22 is illustrated in the diagram of FIG. 4, not drawn to scale relative to FIG. 3. Light emitted from a light source (denoted by the light bulb symbol) reflects from angled portion 34 (a boundary between two mediums), to angled portion 36 (a boundary between two mediums), and to the detector 28 (denoted by the eye symbol). The light transmission is reflected very efficiently through the identification feature 22 and causes a spike of intensity in detector 28. Specifically as illustrated in FIG. 5, a spike 104 is detected, which corresponds to light refracted from refraction element 102 of an anterior ophthalmic surgical cassette 100. The processor 30 calculates the slope of the spike 104, and based on the position of the slope (defined by the detector 28, *e.g.,* linear CCD array, etc.), the cassette type may be determined by the processor 30.

Various aspects of a refraction element, *e.g.,* type of material, the angle of sides, dimensions of sides walls/faces, etc., may be altered in other embodiments to change one of more characteristics of the light refracted, *e.g.,* wavelength, speed, slope, position, intensity, frequency, etc., to indicate a number of distinct cassette types. For example as shown in FIG. 6, an identification feature 200 includes different dimensions, as compared to the identification feature 22, to indicate a cassette type different than the cassette type of FIG. 1. Accordingly, because the aspects of a refraction element may be varied to provide any number of unique spikes, numerous different cassette types may be indicated by an identification feature, including a refraction element.

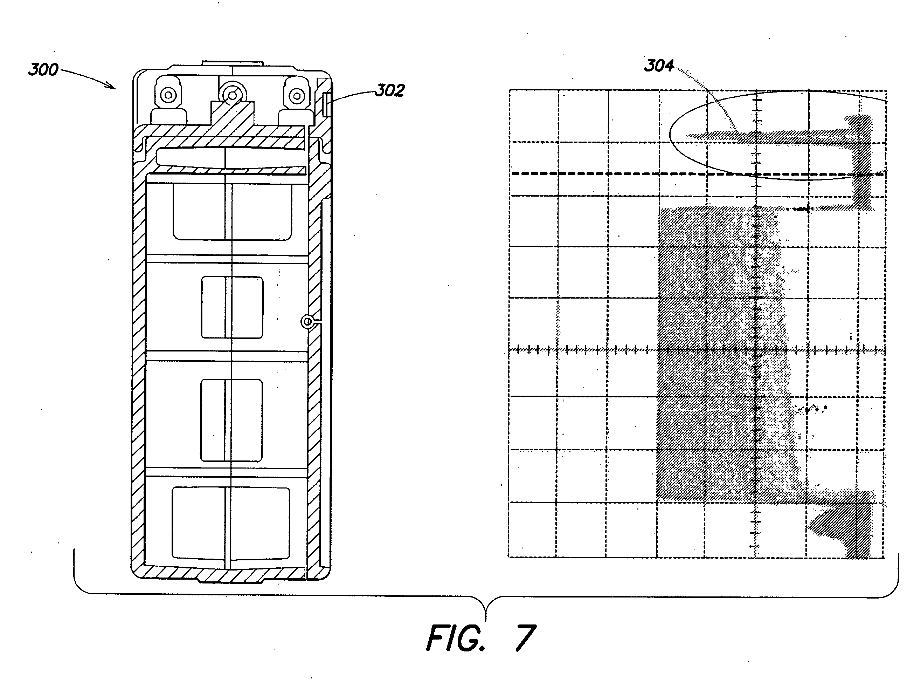

Referring again to FIG. 5 as shown, the spike 104 is sufficiently distinguished to be reliably detected by a detector of an ophthalmic surgical console. In other embodiments, one or more surfaces of an ophthalmic surgical cassette may be treated to minimize the refraction and/or reflection of light from remaining surfaces of an ophthalmic surgical cassette. Specifically as illustrated in FIG. 7, an anterior ophthalmic surgical cassette 300, includes multiple treated surfaces as is well known in the art, to minimize refraction and/or reflection of light (for example, surfaces surrounding feature 302 could be painted black). Accordingly, a spike 304 from an identification feature 302, included in the ophthalmic surgical cassette 300, is easily detectable by an ophthalmic surgical console. It should be appreciated that while the surface treatment of areas of an ophthalmic surgical cassette and manifold surrounding feature 302 may increase the accuracy of detecting an identification feature, positioning an identification feature on a manifold base of the ophthalmic surgical cassette provides sufficient differentiation of a spike, as shown in FIG. 5. Alternatively for example, an identification feature included in a container may require surface treatment to sufficiently differentiate a spike refracted from the identification feature from one or more other refracted/reflected light waveforms in the level sensing portion of a cassette. Surface treatments reduce/eliminate refraction and/or reflection of a container and/or other components of an ophthalmic surgical cassette providing an additional step in manufacturing, thereby adding cost.

Referring again to FIG. 1, the ophthalmic surgical cassette 10 includes a fluid level indicator 38. The fluid level indicator 38 includes a prism, which refracts light similar to the illustrated refraction of FIG. 4. Light is refracted in a first manner above a top surface of aspirant fluid and/or tissue in the ophthalmic surgical cassette, and in a second manner below the top surface of aspirant fluid and/or tissue in the ophthalmic surgical cassette. Accordingly, depending on a detector included in an ophthalmic surgical console, the fluid level indicator 38 may simply indicate when the aspirant fluid and/or tissue has reached a pre-determined point, *e.g.,* full, etc., or when aspirant fluid and/or tissue reaches various other levels within an ophthalmic surgical cassette. Further as shown in FIG. 1, the fluid level indicator 38 is adjacent to and/or inline with the identification feature 22. In this manner, a single detector, *e.g.,* an array of photodetectors, etc., may be employed to detect the identification feature 22, and a level of aspirant fluid and/or tissue within the ophthalmic surgical cassette 10. It should be appreciated that an identification feature may be disposed differently on a manifold base of an ophthalmic surgical cassette in other embodiments of the present disclosure.

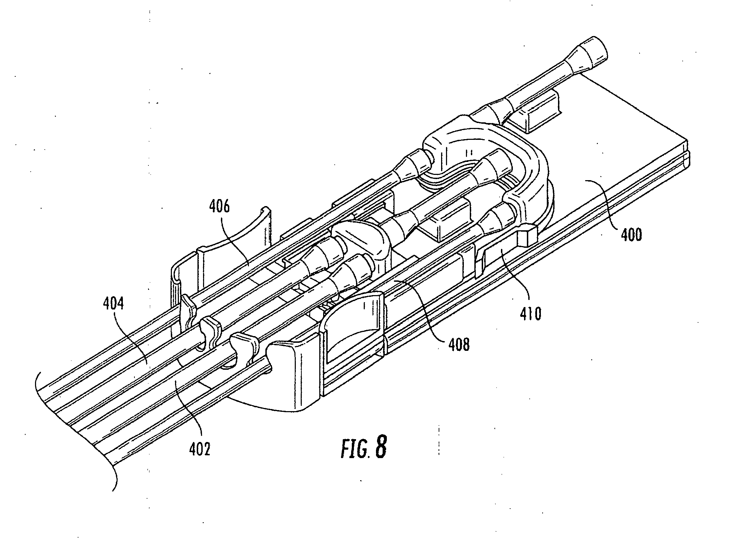

According to other embodiments of the present disclosure, a manifold base may be configured for more than one type of ophthalmic surgical procedure. FIG. 8 illustrates a manifold base 400 to be removeably coupled to a rigid-walled container for one or more posterior ophthalmic surgical procedures. The manifold base 400 includes an inlet irrigation tube 402 for coupling an irrigation source, and outlet irrigation tube 404 for providing irrigation to a surgical site, and an aspiration tube 406 for coupling an ophthalmic surgical handpiece. The manifold base 400 also includes an auxiliary aspiration tube 408 for coupling a second ophthalmic surgical handpiece or other ophthalmic surgical equipment configured to provide aspiration. In various embodiments, an auxiliary aspiration tube 408 may be included so that a surgeon does not have to change aspiration tubes between multiple ophthalmic surgical handpieces during an ophthalmic surgical procedure. Further, the manifold base 400 includes an identification feature 410, similar to the refraction element described above. The identification feature 410 provides an indication of a cassette type for a posterior ophthalmic surgical procedure. Because the cassette type is posterior, an ophthalmic surgical cassette, including the manifold base 400 installed in an ophthalmic surgery pump system, may cause surgical functions for anterior procedures to be disabled.

Alternatively if a cassette type is combined, an ophthalmic surgery pump system may not disable any surgical routines for anterior and/or posterior ophthalmic surgical procedures. In at least one embodiment, an ophthalmic surgical cassette with a cassette type of posterior or combined may not disable surgical functions/routines associated with the other of posterior and combined.

Although several aspects of the present disclosure have been described above with reference to ophthalmic surgical cassettes, it should be understood that various aspects of the present disclosure are not limited to ophthalmic surgical cassettes, and can be applied to a variety of other ophthalmic surgical systems, devices, and methods.

By implementing any or all of the teachings described above, a number of benefits and advantages can be attained, including improved reliability, reduced down time, elimination or reduction of redundant components or systems, avoiding unnecessary or premature replacement of components or systems, and a reduction in overall system and operating costs.

## Claims

1. An ophthalmic surgery pump system (24) for use during an ophthalmic surgery procedure, the ophthalmic surgery pump system comprising:
an ophthalmic surgical cassette (10) including a rigid-walled container (12) and a manifold base (14, 400) removeably attached to the rigid-walled containe (12), the manifold base (14, 400) including an irrigation tube (16, 402, 404), an aspiration tube (18, 406), and an identification feature (22, 410) for indicating a cassette type; and
an ophthalmic surgical console (26), including a detector (28) disposed adjacent to the ophthalmic surgical cassette (10) for detecting the identification feature (22, 410) and a processor (30) operably coupled to the detector for enabling, based on the cassette type, at least one surgical function,
wherein the identification feature (22, 410) includes a refraction element,
wherein the detector (28) includes a linear CCD array,
wherein the ophthalmic surgical cassette (10) includes a fluid level indicator (38) disposed inline with the identification feature (22, 410), and
wherein the linear CCD array extends adjacent to the identification feature (22, 410) and the fluid level indicator (38), such that the identification feature (22, 410) and the fluid level indicator (38) are detectable by the linear CCD array.

2. The ophthalmic surgery pump system of claim 1, wherein the processor (30) is configured to disable at least one surgical function of the ophthalmic surgical console based on the cassette type.

3. The ophthalmic surgery pump system of claim 1 or 2, wherein the manifold base (14, 400) includes an auxiliary aspiration tube (408).

## Patentansprüche

1. Augenchirurgisches Pumpsystem (24) zum Gebrauch während eines augenchirurgischen Eingriffs, wobei das augenchirurgische Pumpsystem aufweist:
eine augenchirurgische Kassette (10) mit einem festwandigen Behälter (12) und
einer Verteilerbasis (14, 400), die am festwandigen Behälter (12) entfernbar angebracht ist, wobei die Verteilerbasis (14, 400) einen Spülschlauch (16, 402, 404), einen Absaugschlauch (18, 406) und ein Identifizierungsmerkmal (22, 410) zum Anzeigen eines Kassettentyps aufweist; und
eine augenchirurgische Konsole (26) mit einem benachbart zur augenchirurgischen Kassette (10) angeordneten Detektor (28) zum Detektieren des Identifizierungsmerkmals (22, 410) und einem mit dem Detektor wirkungsmäßig gekoppelten Prozessor (30) zum Ermöglichen mindestens einer chirurgischen Funktion auf der Grundlage des Kassettentyps,
wobei das Identifizierungsmerkmal (22, 410) ein Brechungselement aufweist,
wobei der Detektor (28) ein CCD-Zeilenarray aufweist,
wobei die augenchirurgische Kassette (10) eine Flüssigkeitsstandanzeige (38) aufweist, die in Reihe mit dem Identifizierungsmerkmal (22, 410) angeordnet ist, und
wobei sich das CCD-Zeilenarray benachbart zum Identifizierungsmerkmal (22, 410) und zur Flüssigkeitsstandanzeige (38) so erstreckt, dass das Identifizierungsmerkmal (22, 410) und die Flüssigkeitsstandanzeige (38) durch das CCD-Zeilenarray detektierbar sind.

2. Augenchirurgisches Pumpsystem nach Anspruch 1, wobei der Prozessor (30) so konfiguriert ist, dass er mindestens eine chirurgische Funktion der augenchirurgischen Konsole auf der Grundlage des Kassettentyps deaktiviert.

3. Augenchirurgisches Pumpsystem nach Anspruch 1 oder 2, wobei die Verteilerbasis (14, 400) einen Hilfsabsaugschlauch (408) aufweist.

## Revendications

1. Système de pompe ophtalmique chirurgicale (24) utilisé pendant une opération de chirurgie ophtalmique, ledit de pompe ophtalmique chirurgicale comprenant :
une cassette chirurgicale ophtalmique (10) comportant un conteneur à paroi rigide (12) et une base de collecteur (14, 400) fixée de manière amovible au conteneur à paroi rigide (12), ladite base de collecteur (14, 400) comportant un tuyau d'irrigation (16, 402, 404), un tuyau d'aspiration (18, 406) et un élément d'identification (22, 410) pour indiquer un type de cassette ; et
une console chirurgicale ophtalmique (26) comportant un détecteur (28) adjacent à la cassette chirurgicale ophtalmique (10) pour la détection de l'élément d'identification (22, 410), et un processeur (30) fonctionnellement couplé au détecteur pour permettre au moins une fonction chirurgicale sur la base du type de cassette,
l'élément d'identification (22, 410) comportant un élément de réfraction,
le détecteur (28) comportant une matrice CCD linéaire,
la cassette chirurgicale ophtalmique (10) comportant un indicateur de niveau de fluide (38) disposé en ligne avec l'élément d'identification (22, 410), et
la matrice CCD linéaire s'étendant adjacente à l'élément d'identification (22, 410) et à l'indicateur de niveau de fluide (38) de manière à rendre l'élément d'identification (22, 410) et l'indicateur de niveau de fluide (38) détectables par la matrice CCD linéaire.

2. Système de pompe ophtalmique chirurgicale selon la revendication 1, où le processeur (30) est prévu pour désactiver au moins une fonction chirurgicale de la console ophtalmique chirurgicale sur la base du type de cassette.

3. Système de pompe ophtalmique chirurgicale selon la revendication 1 ou la revendication 2, où la base de collecteur (14, 400) comporte un tuyau auxiliaire d'aspiration (408).
